# EUROPEAN PATENT APPLICATION

(11) **EP 3 456 816 A1**
(43) Date of publication of application: **20.03.2019**
(21) Application number: 17796106.7
(22) Date of filing: 08.05.2017
(51) Int. Cl.: C12N 5/071, C12N 5/0735, C12N 5/074, C12N 5/0789

(54) **MEDIUM, ADDITIVE FOR ALBUMIN-FREE MEDIUM, AND METHOD FOR CULTURING PLURIPOTENT STEM CELLS**

(30) Priority: 09.05.2016 JP 2016093922
(71) Applicant: KYOWA HAKKO BIO CO., LTD., Chiyoda-ku Tokyo 100-8185 (JP)
(72) Inventor: FURUE Miho, Ibaraki-shi Osaka 567-0085 (JP); YANAGIHARA Kana, Tokyo 100-8185 (JP); SHOJI Shinichiro, Tokyo 100-8185 (JP); TSUKAHARA Masayoshi, Tokyo 100-8185 (JP)
(74) Representative: Vossius & Partner Patentanwälte Rechtsanwälte mbB
(86) International application number: PCT/JP2017/017424
(87) International publication number: WO 2017/195745

(57) **Abstract**

An object of the present invention is to provide an albumin-free medium for serum-free culture of pluripotent stem cells capable of maintaining the undifferentiated state and the pluripotency (pluripotent ability) of the pluripotent stem cells, an additive for an albumin-free medium, and a culture method. The albumin-free medium for serum-free culture of pluripotent stem cells, the additive for an albumin-free medium, and a medium used for the culture method of the invention are characterized by containing at least one selected from the group consisting of a Pluronic nonionic surfactant, an animal-based hydrolysate, and a nonanimal-based hydrolysate.

## Description

### Technical Field

The present invention relates to a medium for serum-free culture of pluripotent stem cells. More particularly, the invention relates to a medium for stably performing serum-free culture of pluripotent stem cells which proliferate while maintaining an undifferentiated state and pluripotency (hereinafter also referred to as "pluripotent ability").

Further, the invention relates to an additive for an albumin-free medium for stably performing serum-free culture of pluripotent stem cells which proliferate while maintaining an undifferentiated state and pluripotency. Still further, the invention relates to a serum-free culture method for proliferating pluripotent stem cells while maintaining the pluripotency of the pluripotent stem cells.

### Background Art

Human artificial pluripotent stem cells (induced pluripotent stem cells: iPS cells) are cells which can be induced by introducing three or four factor genes or the like to somatic cells. Pluripotent stem cells including human iPS cells have infinite proliferation ability, and have an ability to differentiate into almost all the cells in the body (pluripotency). Due to the characteristics, it is expected that pluripotent stem cells are applied to cell therapy or regenerative medicine for diseases which have been difficult to treat so far by artificially producing a tissue from pluripotent stem cells in vitro and transplanting the tissue. Moreover, because it may be possible to use pluripotent stem cells as a replacement for human cells which are difficult to obtain, it is expected that pluripotent stem cells are applied to screening of novel drugs, evaluation of drug efficacy or the like, evaluation of toxicity of chemical substances or the like, elucidation of developmental mechanism or elucidation of association of gene mutations with diseases, etc.

Pluripotent stem cells are desirably cultured in a serum-free medium composed of a known composition. In general, pluripotent stem cells are cultured using a medium supplemented with serum replacement factor KSR (knockout-serum replacement) and fibroblast growth factor-2 (FGF-2) and mouse embryonic tissue-derived fibroblasts or the like as feeder cells. Alternatively, pluripotent stem cells are coated with an extracellular matrix such as Matrigel, vitronectin, laminin, or fibronectin or a recombinant protein thereof or the like and cultured in a medium in which FGF-2 or the like is added to a culture supernatant of feeder cells or the like without using feeder cells. In these culture operations, a component derived from a heterologous species or a heterologous cell is incorporated, and an unknown component is incorporated at an unknown concentration.

KSR is said to be serum-free, but contains animal-derived components with lot-to-lot variations, and the composition thereof is not disclosed. Further, there are lot-to-lot variations also in feeder cells or Matrigel, and there is a concern about the possibility of incorporation of a heterologous cell as well. When a component derived from a heterologous species or a heterologous cell is incorporated in this manner, it becomes a factor to make the culture unstable, and moreover may cause infection with a virus or a pathogen common to humans and animals or may become a heterologous antigen, and therefore is preferably removed from the culture.

Therefore, at present, an extracellular matrix component such as vitronectin, laminin, or fibronectin or a recombinant thereof is used as a scaffolding material for safely culturing pluripotent stem cells, and a chemically defined serum-free medium which is a serum-free medium whose components are all known is used as a medium. As the medium, for example, TeSR1 medium, TeSR-E8 medium, STEMPRO medium, hESF-FX medium, or the like is exemplified.

In a main composition of such a medium, high-glucose containing DMEM (Dulbecco's Modified Eagle's medium) or DMEM/F12 (a medium obtained by mixing DMEM and F12 medium at 1:1) is used as a basal medium, inorganic salts, amino acids, vitamins, saccharides, and minor components are contained, and further, insulin, transferrin, albumin, a lipid group or the like, a growth factor or the like, mercaptoethanol, or the like is added.

Among these medium additive components, albumin, particularly, human-derived albumin or recombinant albumin is expensive, biological activity varies depending on the manufacturer or the origin, which may become a factor to make the medium unstable. Further, the biological activity of bovine-derived albumin also varies depending on the manufacturer or the lot, and a pathogen may be incorporated therein, and in the case of medical application, confirmation of a biological origin is needed, and therefore, bovine-derived albumin becomes expensive. Further, albumin has an action to conjugate an added factor in a medium and reduce the effect thereof, and therefore, when albumin is contained in a medium, it is difficult to detect the effect of the added factor with high sensitivity. Therefore, it is desired to replace albumin with a component other than proteins or remove albumin from a medium.

As the medium in which albumin is replaced with a component other than proteins, the following media are known although they are examples in which a mammalian-derived cell line was cultured.

PTL 1 discloses a medium which is a medium for culturing a mammal-derived cell line continuously producing a recombinant protein and is substantially free of protein components. In PTL 1, a clathrate compound of α-cyclodextrin, an unsaturated fatty acid, and a fat-soluble vitamin is exemplified as an albumin replacement. Further, it is disclosed that a mammal-derived cell line can be cultured also by adding a microemulsion of a fat-soluble factor and Pluronic (registered trademark) F-68 which is a Pluronic nonionic surfactant.

PTL 2 discloses a method for producing a recombinant protein by culturing animal cells in a protein-free medium, and discloses a method for producing a recombinant protein in a medium supplemented with Pluronic (registered trademark) F-68.

PTL 3 discloses the use of polyethylene glycol as an albumin replacement in a medium for culturing animal cells or tissue cells.

In an example of culturing stem cells, as a medium in which albumin is replaced with a component other than proteins, NPL 1 discloses the addition of polyvinyl alcohol as an albumin replacement to a medium for culturing embryonic stem cells (ES cells).

On the other hand, NPL 2 discloses that Pluronic (registered trademark) F-68 accelerates the differentiation of mesenchymal stem cells.

### Citation List

### Patent Literature

PTL 1: Japanese Patent No. 2696001
PTL 2: Japanese Patent No. 4257685
PTL 3: JP-A-2004-135672

### Non Patent Literature

NPL 1: Ludovic Vallier et al, PLoS ONE, volume 4, e6082 (2009)
NPL 2: Dogan et al, International Journal of Nanomedicine 2012: 7 4849-4860

### Summary of Invention

### Technical Problem

However, none of these documents describe or suggest that pluripotent stem cells can be cultured while maintaining the undifferentiated state and the pluripotency of the pluripotent stem cells by adding at least one selected from the group consisting of a Pluronic nonionic surfactant, an animal-based hydrolysate, and a nonanimal-based hydrolysate to a medium which does not contain albumin.

Further, in a conventionally known medium, although pluripotent stem cells can be cultured while maintaining the undifferentiated state and the pluripotency of the pluripotent stem cells, the cost is high and lots are unstable in many cases. In particular, human-derived or recombinant albumin or bovine albumin whose biological origin can be confirmed to be used as a medium component is expensive. In addition, the biological activity varies depending on the manufacturer, the origin, and the transport route, which may become a factor to make the medium unstable.

Therefore, an object of the invention is to provide an albumin-free medium for stably and inexpensively performing serum-free culture of pluripotent stem cells capable of maintaining the undifferentiated state and the pluripotency of the pluripotent stem cells, to provide an additive for an albumin-free medium for stably and inexpensively performing serum-free culture of pluripotent stem cells, and to provide a serum-free culture method for stably and inexpensively proliferating pluripotent stem cells while maintaining the undifferentiated state and the pluripotency of the pluripotent stem cells.

### Solution to Problem

The present inventors found that the above object can be achieved by using at least one selected from the group consisting of a Pluronic nonionic surfactant, an animal-based hydrolysate, and a nonanimal-based hydrolysate, and thus completed the invention.

That is, the present invention relates to the following (1) to (10).
(1) A medium for serum-free culture of pluripotent stem cells, containing at least one selected from the group consisting of a Pluronic nonionic surfactant, an animal-based hydrolysate, and a nonanimal-based hydrolysate, and not containing albumin.
(2) The medium described in (1), further containing at least one of a lipid mixture and water-soluble oleic acid.
(3) The medium described in (1) or (2), wherein the Pluronic nonionic surfactant is Pluronic (registered trademark) F-68.
(4) The medium described in any one of (1) to (3), wherein the nonanimal-based hydrolysate is EX-CELL (registered trademark) CD Hydrolysate Fusion (manufactured by Sigma-Aldrich Co. LLC.).
(5) An additive for an albumin-free medium for serum-free culture of pluripotent stem cells, containing at least one selected from the group consisting of a Pluronic nonionic surfactant, an animal-based hydrolysate, and a nonanimal-based hydrolysate.
(6) The additive for an albumin-free medium described in (5), further containing at least one of a lipid mixture and water-soluble oleic acid.
(7) The additive for an albumin-free medium described in (5) or (6), wherein the nonanimal-based hydrolysate is EX-CELL (registered trademark) CD Hydrolysate Fusion (manufactured by Sigma-Aldrich Co. LLC.).
(8) A method for culturing pluripotent stem cells, comprising culturing pluripotent stem cells in a serum-free medium containing at least one selected from the group consisting of a Pluronic nonionic surfactant, an animal-based hydrolysate, and a nonanimal-based hydrolysate, and not containing albumin, thereby proliferating the pluripotent stem cells while maintaining the pluripotency of the pluripotent stem cells.
(9) The culture method described in (8), wherein the serum-free medium further contains at least one of a lipid mixture and water-soluble oleic acid.
(10) The culture method described in (8) or (9), wherein the nonanimal-based hydrolysate is EX-CELL (registered trademark) CD Hydrolysate Fusion (manufactured by Sigma-Aldrich Co. LLC.).

### Advantageous Effects of Invention

According to the medium, the additive for an albumin-free medium, and the culture method of the invention, by including at least one selected from the group consisting of a Pluronic nonionic surfactant, an animal-based hydrolysate, and a nonanimal-based hydrolysate, pluripotent stem cells can be cultured without adding a serum-derived component, albumin, or a recombinant thereof to the medium, and can be stably and inexpensively proliferated while maintaining the undifferentiated state and the pluripotency of the pluripotent stem cells.

According to the medium, the additive for an albumin-free medium, and the culture method of the invention, pluripotent stem cells can be proliferated by serum-free culture composed of a known composition, they are useful for various researches or regenerative medicine and the like, and eliminate consumers' anxiety generated when using a mammal-derived component such as serum, and can provide highly safe medical treatment.

Further, as described above, albumin has an action to conjugate an added factor and reduce the effect thereof, however, according to the medium and the additive for an albumin-free medium of the invention, each of which does not contain albumin, the effect of the added factor can be detected with high sensitivity. Therefore, the medium and the additive for an albumin-free medium of the invention are useful also for evaluation of drug efficacy or toxicity of a low molecular weight compound or the like or screening or the like.

### Description of Embodiments

### 1. Medium

The invention relates to a medium which contains at least one selected from the group consisting of a Pluronic nonionic surfactant, an animal-based hydrolysate, and a nonanimal-based hydrolysate, and does not contain albumin (hereinafter also referred to as "the medium of the invention").

The medium of the invention is used for performing serum-free culture of pluripotent stem cells, and the "stem cell" as used herein refers to a cell having a self-renewal ability to produce a cell of the same type as itself and a pluripotent ability.

There is a hierarchy of stem cells, and it is known that undifferentiated stem cells at a high level has a high self-renewal ability and also has high pluripotency capable of differentiating into various cell lineages, however, as the level goes down, the cells lose the self-renewal ability and can differentiate only into a specific cell lineage.

Cells to be mainly cultured in the medium of the invention are cells hierarchically located at a relatively high level, in other words, stem cells (pluripotent stem cells) in an undifferentiated state and capable of self-renewal while sufficiently maintaining pluripotency. The origin of the pluripotent stem cells may be a human or a non-human animal.

The pluripotent stem cells refer to stem cells capable of self-renewal and having an ability to differentiate into differentiated cells belonging to three germ layers (ectoderm, mesoderm, and endoderm) (also referred to as "pluripotent ability" or "pluripotency").

Examples of the pluripotent stem cells include embryonic germ cells (EG cells), embryonal carcinoma cells (EC cells), embryonic stem cells (ES cells), induced pluripotent stem cells (iPS cells), and adult pluripotent stem cells (APS cells).

The "pluripotency" as used herein can also be referred to as "pluripotent ability", and means an ability to be able to differentiate into any of differentiated cells belonging to ectoderm, mesoderm, and endoderm, and may also include an ability to differentiate into germ cells here. Incidentally, the differentiated cells are many types of cells constituting tissues or organs in the body.

The "serum-free culture" as used herein means culture without using serum. Therefore, the medium provided herein means culture enabling proliferation of pluripotent stem cells while maintaining the undifferentiated state and the pluripotency of the undifferentiated pluripotent stem cells even without using serum.

The Pluronic nonionic surfactant used in the medium of the invention means a triblock copolymer nonionic surfactant composed of hydrophilic ethylene oxide (EO) and hydrophobic propylene oxide (PO).

Examples of the Pluronic nonionic surfactant include Pluronic (registered trademark) F-61, Pluronic (registered trademark) F-68, Pluronic (registered trademark) F-71, and Pluronic (registered trademark) F-108, and among these, Pluronic (registered trademark) F-68 is preferred.

The content of the Pluronic nonionic surfactant in the medium of the invention is preferably 0.001 mass% or more, more preferably 0.005 mass% or more, further more preferably 0.01 mass% or more, and also preferably 10 mass% or less, more preferably 1 mass% or less, further more preferably 0.5 mass% or less.

By setting the content of the Pluronic nonionic surfactant within the above range, the undifferentiated state and the pluripotency of pluripotent stem cells can be efficiently maintained, and it is effective in adhesion and proliferation of the pluripotent stem cells maintaining the undifferentiated state and the pluripotency. Further, the number of pluripotent stem cells which do not have cytotoxicity and maintain the undifferentiated state and the pluripotency can be increased.

Examples of the animal-based hydrolysate used in the medium of the invention include a meat hydrolysate and a mammal-derived casein hydrolysate such as a milk-derived casein hydrolysate. Examples of the nonanimal-based hydrolysate used in the medium of the invention include soybean, wheat gluten, yeast extract, and vegetable-derived hydrolysates.

All or some of the components of the animal-based hydrolysate or the nonanimal-based hydrolysate may be replaced with those derived from synthesis or fermentation.

Examples of the animal-based hydrolysate in which some components are replaced with those derived from synthesis or fermentation used in the medium of the invention include Casein Yeast Peptone (manufactured by Sigma-Aldrich Co. LLC.). Further, examples of the nonanimal-based hydrolysate include EX-CELL (registered trademark) CD Hydrolysate Fusion (manufactured by Sigma-Aldrich Co. LLC.).

The content of the animal-based hydrolysate or the nonanimal-based hydrolysate in the medium of the invention is preferably 0.0001 mass% or more, more preferably 0.001 mass% or more, further more preferably 0.005 mass% or more, and also preferably 10 mass% or less, more preferably 0.5 mass% or less, further more preferably 0.05 mass% or less.

By setting the content of the animal-based hydrolysate or the nonanimal-based hydrolysate within the above range, the undifferentiated state and the pluripotency of pluripotent stem cells can be efficiently maintained, and it is effective in adhesion and proliferation of the pluripotent stem cells in an undifferentiated state having pluripotency. Further, the number of pluripotent stem cells which have reduced cytotoxicity and maintain the undifferentiated state and the pluripotency can be increased.

In the medium of the invention, at least one selected from the group consisting of a Pluronic nonionic surfactant, an animal-based hydrolysate, and a nonanimal-based hydrolysate is contained, and two or more types among these may also be used in combination.

The medium of the invention does not contain albumin. Albumin also includes naturally derived albumin and a recombinant thereof. The term "not contain" as used herein may be "substantially not contain". The term "substantially not contain" includes to contain to such an extent that an adverse effect is not caused.

The medium of the invention may further contain at least one of a lipid mixture and water-soluble oleic acid. The "lipid mixture" as used herein means a defined (for example, chemically defined) lipid mixture required for culturing pluripotent stem cells. The lipid mixture is generally added to a medium which does not contain serum or a serum replacement, and generally replaces a lipid to be added to a preparation of serum or a serum replacement by doing this.

Examples of the lipid mixture include Chemically Defined Lipid Concentrate (Cat No. 11905031, manufactured by Thermo Fisher Scientific, Inc.).

The content of the lipid mixture in the medium of the invention is preferably 0.00001 mass% or more, more preferably 0.00005 mass% or more, further more preferably 0.0001 mass% or more, and also preferably 10 mass% or less, more preferably 5 mass% or less, further more preferably 2 mass% or less. By setting the content of the lipid mixture within the above range, the number of cells maintaining the undifferentiated state and the pluripotency is increased, and the effect thereof is comparable to that when albumin is added. Further, the cells which do not have cytotoxicity and maintain the undifferentiated state and the pluripotency are increased.

Further, when the medium of the invention contains water-soluble oleic acid, the content thereof is preferably 0.01 ng/mL or more, more preferably 0.1 ng/mL or more, further more preferably 1 ng/mL or more, and also preferably 10 µg/mL or less, more preferably 1 µg/mL or less, further more preferably 100 ng/mL or less. By setting the content of water-soluble oleic acid within the above range, the number of cells maintaining the undifferentiated state and the pluripotency is increased, and the effect thereof is comparable to that when albumin is added. Further, cytotoxicity of water-soluble oleic acid disappears, and the number of cells maintaining the undifferentiated state and the pluripotency is increased.

The medium of the invention may further contain medium basal components. The medium basal components generally include a cell-assimilable carbon source, a digestible nitrogen source, and inorganic salts. Specific examples thereof include inorganic salts, amino acids, glucose, and vitamins. Further, in the medium basal components, a minor active substance such as a minor nutrient enhancer or precursor may be further blended as needed.

As such medium basal components, a basal medium known to those skilled in the art can be used, and examples thereof include MEM (Minimum Essential Medium), BME (Basal Medium Eagle), DMEM (Dulbecco's Modified Eagle Medium), EMEM (Eagle's minimal essential medium), IMDM (Iscove's Modified Dulbecco's Medium), GMEM (Glas-gow's MEM), F12 (Ham's F12 Medium), DMEM/F12, RPMI1640, RD, BMOC-3 (Brinster's BMOC-3 Medium), CMRL-1066, L-15 medium (Leibovitz's L-15 medium), McCoy's 5A, Media 199, MEM αMedia, MCDB105, MCDB131, MCDB153, MCDB201, and Williams' medium E and ESF.

Further, to the medium of the invention, a nucleic acid, a nonessential amino acid, a reducing agent, or the like may be added as needed. Further, if necessary, an arbitrary component such as a pH adjusting agent, a buffer component, a humectant, a preservative, or a viscosity adjusting agent can also be used.

### 2. Additive for Albumin-Free Medium

The invention relates to an additive for an albumin-free medium for serum-free culture of pluripotent stem cells, characterized by containing at least one selected from the group consisting of a Pluronic nonionic surfactant, an animal-based hydrolysate, and a nonanimal-based hydrolysate (hereinafter also referred to as "the additive of the invention").

The additive of the invention contains at least one selected from the group consisting of a Pluronic nonionic surfactant, an animal-based hydrolysate, and a nonanimal-based hydrolysate, and can also use two or more types among these in combination.

The additive of the invention can be used for serum-free culture of pluripotent stem cells while maintaining the undifferentiated state and the pluripotency of the pluripotent stem cells by being added to conventionally known medium basal components not containing albumin.

As described above, the additive of the invention is an additive in which at least one selected from the group consisting of a Pluronic nonionic surfactant, an animal-based hydrolysate, and a nonanimal-based hydrolysate is added to conventionally known medium basal components.

As the Pluronic nonionic surfactant, the animal-based hydrolysate, and the nonanimal-based hydrolysate, those described above can be used.

As the content of the Pluronic nonionic surfactant in the additive of the invention, the concentration thereof when it is mixed in the above-mentioned medium basal components to form a medium is preferably 0.001 mass% or more, more preferably 0.005 mass% or more, further more preferably 0.01 mass% or more, and also preferably 10 mass% or less, more preferably 1 mass% or less, further more preferably 0.5 mass% or less.

As the content of the animal-based hydrolysate or the nonanimal-based hydrolysate in the additive of the invention, the concentration thereof when it is mixed in the above-mentioned medium basal components to form a medium is preferably 0.0001 mass% or more, more preferably 0.001 mass% or more, further more preferably 0.005 mass% or more, and also preferably 10 mass% or less, more preferably 0.5 mass% or less, further more preferably 0.05 mass% or less.

The additive of the invention may further contain at least one of a lipid mixture and water-soluble oleic acid. As the lipid mixture, those described above can be used, and the content thereof when preparing a medium is preferably 0.00001 mass% or more, more preferably 0.00005 mass% or more, further more preferably 0.0001 mass% or more, and also preferably 10 mass% or less, more preferably 5 mass% or less, further more preferably 2 mass% or less.

Further, when the additive of the invention contains water-soluble oleic acid, the content thereof when preparing a medium is preferably 0.01 ng/mL or more, more preferably 0.1 ng/mL or more, further more preferably 1 ng/mL or more, and also preferably 10 µg/mL or less, more preferably 1 µg/mL or less, further more preferably 100 ng/mL or less.

Examples of the method for confirming whether or not the cells cultured in the medium of the invention or a medium produced using the additive of the invention are cells maintaining the undifferentiated state and the pluripotency include a method for determining through the detection of expression of an undifferentiation marker and/or the non-detection of expression of a differentiation marker using an antibody to a marker (protein), a method for determining through the detection of expression of a marker (gene), a method through the observation of the morphological features of the cells, and a method for determining whether or not the cells can exhibit an ability to differentiate into specific cells through stimulation by a specific differentiation-inducing factor.

In the methods for determining using a marker, it can be determined that the cells cultured in the medium of the invention or a medium produced using the additive of the invention maintain the undifferentiated state and the pluripotency when an undifferentiation marker such as NANOG, OCT3/4, SSEA-3, SSEA-4, TRA-2-54, TRA-1-60, TRA-1-80, CD90, or alkaline phosphatase is expressed.

In the case where the expression of the undifferentiation marker and/or the differentiation marker is confirmed at the protein level, it can be confirmed, for example, using a specific antibody to the marker by flow cytometry, immunostaining, ELISA, or the like. In the case where the expression of the marker is confirmed at the gene level, it can be confirmed, for example, by RT-PCR using a specific primer pair for the marker gene, northern blotting or transcriptome analysis using a specific probe, or the like.

Specific examples of the method for confirming whether or not a marker is expressed by flow cytometry include the following method. The cells cultured in the medium of the invention or a medium produced using the additive of the invention are suspended in 1 mL of 10% goat serum for 30 minutes and then centrifuged. Subsequently, the cells are incubated for 30 hours together with a mouse antibody to the target marker protein. Then, the cultured cells are washed three times with PBS containing 1% goat serum, reacted with a fluorescent label (Alexa Fluor)-conjugated goat anti-mouse IgG antibody for 30 minutes and washed three times with PBS containing 1% goat serum, and the resuspended cells can be subjected to determination using an appropriate flow cytometry device.

Specific examples of the method for confirming whether or not a marker is expressed by immunostaining include the following method. The cells cultured in the medium of the invention or a medium produced using the additive of the invention are fixed with 4% paraformaldehyde (PFA) in PBS and then washed more than once with PBS. After blocking with 10% goat serum, the cells are immunostained using a mouse antibody to the target marker protein, reacted with fluorescent label (Alexa Fluor)-conjugated goat anti-mouse IgG and subjected to determination by observation with a fluorescence microscope. When the marker protein is in the cytoplasm, the permeability of the cultured cells is increased using Triton X or the like, and then, immunostaining is performed using the antibody.

Specific examples of the method for confirming whether or not alkaline phosphatase is expressed include the following method. The cells cultured in the medium of the invention or a medium produced using the additive of the invention are fixed for 5 minutes with 4.5 mM citric acid, 2.25 mM sodium citrate, 3 mM sodium chloride, 65% methanol, and 4% paraformaldehyde and washed with distilled water, and then visualization of alkaline phosphatase, so-called alkaline phosphatase staining can be performed using an appropriate kit such as FastRed substrate kit (manufactured by Sigma, Inc.).

### 3. Method for Culturing Pluripotent Stem Cells

The invention relates to a method for culturing pluripotent stem cells, characterized by culturing pluripotent stem cells in a serum-free medium containing at least one selected from the group consisting of a Pluronic nonionic surfactant, an animal-based hydrolysate, and a nonanimal-based hydrolysate, and not containing albumin, thereby proliferating the pluripotent stem cells while maintaining the pluripotency of the pluripotent stem cells (hereinafter also referred to as "the culture method of the invention").

As the medium used in the culture method of the invention, the medium described in the above 1 can be used. As described in the above 1, the medium used in the culture method of the invention contains at least one selected from the group consisting of a Pluronic nonionic surfactant, an animal-based hydrolysate, and a nonanimal-based hydrolysate, and does not contain albumin.

The content of the Pluronic nonionic surfactant in the medium used in the culture method of the invention is preferably 0.001 mass% or more, more preferably 0.005 mass% or more, further more preferably 0.01 mass% or more, and also preferably 10 mass% or less, more preferably 1 mass% or less, further more preferably 0.5 mass% or less.

The content of the animal-based hydrolysate or the nonanimal-based hydrolysate in the medium used in the culture method of the invention is preferably 0.0001 mass% or more, more preferably 0.001 mass% or more, further more preferably 0.005 mass% or more, and also preferably 10 mass% or less, more preferably 0.5 mass% or less, further more preferably 0.05 mass% or less.

The medium used in the culture method of the invention may further contain at least one of a lipid mixture and water-soluble oleic acid. As the lipid mixture, those described above can be used, and the content thereof when preparing the medium is preferably 0.00001 mass% or more, more preferably 0.00005 mass% or more, further more preferably 0.0001 mass% or more, and also preferably 10 mass% or less, more preferably 5 mass% or less, further more preferably 2 mass% or less.

Further, when the medium used in the culture method of the invention contains water-soluble oleic acid, the content thereof when preparing the medium is preferably 0.01 ng/mL or more, more preferably 0.1 ng/mL or more, further more preferably 1 ng/mL or more, and also preferably 10 µg/mL or less, more preferably 1 µg/mL or less, further more preferably 100 ng/mL or less.

It is preferred to perform the culture in the culture method of the invention at generally 33 to 40°C, preferably 34 to 39°C, more preferably 36 to 37°C, in the presence of carbon dioxide at generally 1 to 20%, preferably 3 to 15%, more preferably 8 to 10%, at a relative humidity of generally 75% or more, preferably 85% or more, more preferably 95% or more, particularly preferably 100%.

Examples of the method for confirming whether or not the cells cultured by the culture method of the invention maintain the undifferentiated state and the pluripotency include the method described in the above 2.

### Examples

Next, the invention is described in more detail referring to Examples, but the invention is not limited by these Examples.

### [Example 1]

In a known medium (mESF Basal Medium, manufactured by Wako Pure Chemical Industries, Ltd.), 10 µg/mL insulin (manufactured by Sigma-Aldrich Co. LLC.), 5 µg/mL apotransferrin (manufactured by Sigma-Aldrich Co. LLC.), and 0.01 mass% EX-CELL (registered trademark) CD Hydrolysate Fusion (manufactured by Sigma-Aldrich Co. LLC.) were added and dissolved, and the resulting material was used as a medium. As the cells, human iPS cell 253G1 strain (established by Center for iPS Cell Research and Application, Kyoto University and obtained from iPS Academia Japan, Inc.) was used.

The above medium was added to a 24-well plate for cell culture (manufactured by Sumitomo Bakelite Company Limited) at 1 mL/well, and the above cells were cultured in the presence of 10% CO₂ at 37°C for 3 days. The cultured cells were subjected to nuclear staining with 4',6-diamidino-2-phenylindole (DAPI), and the number of stained cells was counted using IN Cell Analyzer (manufactured by GE Healthcare). Further, OCT3/4 protein in cells expressing an undifferentiation marker gene OCT3/4 was immunostained, and the number of cells was counted in the same manner.

As a result, in the case where 0.01 mass% EX-CELL (registered trademark) CD Hydrolysate Fusion was added to the medium, the number of cells stained with DAPI increased to 1.28 times, and the number of cells expressing OCT3/4 increased to 1.33 times as compared with the case where EX-CELL (registered trademark) CD Hydrolysate Fusion was not added. Further, the ratio of the number of cells expressing OCT3/4 to the number of cells stained with DAPI was 74.4% in the test group in which EX-CELL (registered trademark) CD Hydrolysate Fusion was not added to the medium, and 77.2% in the test group in which EX-CELL (registered trademark) CD Hydrolysate Fusion was added to the medium, which were comparable to each other.

From these results, it was found that by culturing pluripotent stem cells in a serum-free medium which contains a nonanimal-based hydrolysate and does not contain albumin, the pluripotent stem cells can be proliferated while maintaining the undifferentiated state and the pluripotency of the pluripotent stem cells.

### [Example 2]

The number of cells stained with DAPI and the number of cells expressing OCT3/4 were counted in the same manner as in Example 1 except that 0.05 mass% Pluronic (registered trademark) F-68 (manufactured by Thermo Fisher Scientific, Inc.) was used in place of EX-CELL (registered trademark) CD Hydrolysate Fusion.

As a result, in the case where 0.05 mass% Pluronic (registered trademark) F-68 was added to the medium, the number of cells stained with DAPI increased to 1.22 times, and the number of cells expressing OCT3/4 increased to 1.20 times as compared with the case where Pluronic (registered trademark) F-68 was not added. Further, the ratio of the number of cells expressing OCT3/4 to the number of cells stained with DAPI was 74.4% in the test group in which Pluronic (registered trademark) F-68 was not added to the medium, and 73.0% in the test group in which Pluronic (registered trademark) F-68 was added to the medium, which were comparable to each other.

As described above, Pluronic (registered trademark) F-68 has been reported to promote the differentiation of mesenchymal stem cells (Dogan et al., International Journal of Nanomedicine 2012: 7 4849-4860). Therefore, the results that by culturing iPS cells which are pluripotent stem cells in a serum-free culture which contains Pluronic (registered trademark) F-68 and does not contain albumin, the differentiation of the iPS cells is suppressed were not expected.

From these results, it was found that by culturing pluripotent stem cells in a serum-free medium which contains a Pluronic nonionic surfactant and does not contain albumin, the pluripotent stem cells can be proliferated while maintaining the undifferentiated state and the pluripotency of the pluripotent stem cells.

### [Example 3]

The number of cells stained with DAPI and the number of cells expressing OCT3/4 were counted in the same manner as in Example 1 except that a medium obtained by adding 0.2 mass% of a lipid mixture (Chemically Defined Lipid Concentrate, Cat No. 11905031, manufactured by Thermo Fisher Scientific, Inc.) and 0.05 mass% Pluronic (registered trademark) F-68 (manufactured by Thermo Fisher Scientific, Inc.) in place of EX-CELL (registered trademark) CD Hydrolysate Fusion was used.

As a result, when adding Pluronic (registered trademark) F-68 and the lipid mixture to the medium, the number of cells stained with DAPI and the number of cells expressing OCT3/4 both increased to 1.09 times as compared with the case where only Pluronic (registered trademark) F-68 was added. Further, the ratio of the number of cells expressing OCT3/4 to the number of cells stained with DAPI was 99.1% in the test group in which only Pluronic (registered trademark) F-68 was added, and 99.0% in the test group in which Pluronic (registered trademark) F-68 and the lipid mixture were added, which were comparable to each other.

From these results, it was found that by culturing pluripotent stem cells in a medium obtained by further adding a lipid mixture to a serum-free medium which does not contain albumin in addition to a Pluronic nonionic surfactant, the effect of proliferating the pluripotent stem cells while maintaining the undifferentiated state and the pluripotency of the pluripotent stem cells is further enhanced as compared with the case where a Pluronic nonionic surfactant is added alone to the medium.

### [Example 4]

The number of cells stained with DAPI and the number of cells expressing OCT3/4 were counted in the same manner as in Example 1 except that a medium obtained by adding 0.05 mass% Pluronic (registered trademark) F-68 (manufactured by Thermo Fisher Scientific, Inc.) and 0.05 mass% EX-CELL (registered trademark) CD Hydrolysate Fusion (manufactured by Sigma-Aldrich Co. LLC.) in place of 0.01 mass% EX-CELL (registered trademark) CD Hydrolysate Fusion was used.

As a result, when adding Pluronic (registered trademark) F-68 and EX-CELL (registered trademark) CD Hydrolysate Fusion to the medium, the number of cells stained with DAPI and the number of cells expressing OCT3/4 both increased to 1.09 times as compared with the case where Pluronic (registered trademark) F-68 was added alone to the medium. Further, the ratio of the number of cells expressing OCT3/4 to the number of cells stained with DAPI was 99.1% in both the test group in which only Pluronic (registered trademark) F-68 was added, and the test group in which Pluronic (registered trademark) F-68 and EX-CELL (registered trademark) CD Hydrolysate Fusion were added, which were comparable to each other.

Further, also in comparison with the test group in which EX-CELL (registered trademark) CD Hydrolysate Fusion was added alone to the medium, the number of cells stained with DAPI and the number of cells expressing OCT3/4 both increased in the test group in which Pluronic (registered trademark) F-68 and EX-CELL (registered trademark) CD Hydrolysate Fusion were added.

From these results, it was found that by culturing pluripotent stem cells in a medium obtained by adding a Pluronic nonionic surfactant and a nonanimal-based hydrolysate to a serum-free medium which does not contain albumin, the effect of proliferating the pluripotent stem cells while maintaining the undifferentiated state and the pluripotency of the pluripotent stem cells is further enhanced as compared with the case where each of a Pluronic nonionic surfactant and a nonanimal-based hydrolysate is added alone to the medium.

### [Example 5]

The number of cells stained with DAPI and the number of cells expressing OCT3/4 were counted in the same manner as in Example 1 except that a medium obtained by adding 0.05 mass% Pluronic (registered trademark) F-68 (manufactured by Thermo Fisher Scientific, Inc.) and 10 ng/mL water-soluble oleic acid in place of EX-CELL (registered trademark) CD Hydrolysate Fusion was used.

As a result, when adding Pluronic (registered trademark) F-68 and water-soluble oleic acid to the medium, the number of cells stained with DAPI and the number of cells expressing OCT3/4 both increased to 1.17 times as compared with the case where Pluronic (registered trademark) F-68 was added alone. Further, the ratio of the number of cells expressing OCT3/4 to the number of cells stained with DAPI was 99.1% in both the test group in which only Pluronic (registered trademark) F-68 was added, and the test group in which Pluronic (registered trademark) F-68 and water-soluble oleic acid were added, which were comparable to each other.

From these results, it was found that by culturing pluripotent stem cells in a medium obtained by further adding water-soluble oleic acid to a serum-free medium which does not contain albumin in addition to a Pluronic nonionic surfactant, the effect of proliferating the pluripotent stem cells while maintaining the undifferentiated state and the pluripotency of the pluripotent stem cells is further enhanced as compared with the case where a Pluronic nonionic surfactant is added alone to the medium.

From the above results, it was found that by culturing pluripotent stem cells in a serum-free medium which contains at least one selected from the group consisting of a Pluronic nonionic surfactant, an animal-based hydrolysate, and a nonanimal-based hydrolysate, and does not contain albumin, the pluripotent stem cells can be proliferated while maintaining the undifferentiated state and the pluripotency of the pluripotent stem cells. Further, it was found that by further adding at least one of a lipid mixture and water-soluble oleic acid to the medium, the effect of proliferating the pluripotent stem cells while maintaining the undifferentiated state and the pluripotency of the pluripotent stem cells can be further enhanced.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2016-093922) filed on May 9, 2016 and the entire contents of which are incorporated herein by reference. Further, all references cited herein are incorporated herein by reference in their entirety.

## Claims

1. A medium for serum-free culture of pluripotent stem cells, containing at least one selected from the group consisting of a Pluronic nonionic surfactant, an animal-based hydrolysate, and a nonanimal-based hydrolysate, and not containing albumin.

2. The medium according to claim 1, further containing at least one of a lipid mixture and water-soluble oleic acid.

3. The medium according to claim 1 or 2, wherein the Pluronic nonionic surfactant is Pluronic (registered trademark) F-68.

4. The medium according to any one of claims 1 to 3, wherein the nonanimal-based hydrolysate is EX-CELL (registered trademark) CD Hydrolysate Fusion (manufactured by Sigma-Aldrich Co. LLC.).

5. An additive for an albumin-free medium for serum-free culture of pluripotent stem cells, containing at least one selected from the group consisting of a Pluronic nonionic surfactant, an animal-based hydrolysate, and a nonanimal-based hydrolysate.

6. The additive for an albumin-free medium according to claim 5, further containing at least one of a lipid mixture and water-soluble oleic acid.

7. The additive for an albumin-free medium according to claim 5 or 6, wherein the nonanimal-based hydrolysate is EX-CELL (registered trademark) CD Hydrolysate Fusion (manufactured by Sigma-Aldrich Co. LLC.).

8. A method for culturing pluripotent stem cells, comprising culturing pluripotent stem cells in a serum-free medium containing at least one selected from the group consisting of a Pluronic nonionic surfactant, an animal-based hydrolysate, and a nonanimal-based hydrolysate, and not containing albumin, thereby proliferating the pluripotent stem cells while maintaining the pluripotency of the pluripotent stem cells.

9. The culture method according to claim 8, wherein the serum-free medium further contains at least one of a lipid mixture and water-soluble oleic acid.

10. The culture method according to claim 8 or 9, wherein the nonanimal-based hydrolysate is EX-CELL (registered trademark) CD Hydrolysate Fusion (manufactured by Sigma-Aldrich Co. LLC.).
